# EUROPÄISCHE PATENTSCHRIFT

(11) **EP 2 775 285 B1**
(45) Veröffentlichungstag und Bekanntmachung des Hinweises auf die Patenterteilung: **09.08.2017**
(21) Anmeldenummer: 14171474.1
(22) Anmeldetag: 12.06.2008
(51) Int. Cl.: G01N 1/22, G01N 33/20

(54) **Verfahren zum Messen eines Gasgehaltes in einer Metallschmelze**
Method for measuring a gas content in a metal melt
Procédé de mesure d'une teneur en gaz dans un métal en fusion

(30) Priorität: 10.07.2007 DE 102007032436
(43) Veröffentlichungstag der Anmeldung: 10.09.2014
(62) Teilanmeldung aus: 08010689.1
(73) Patentinhaber: Heraeus Electro-Nite International N.V., 3530 Houthalen (BE)
(72) Erfinder: Gerits, Erik, 3600 Genk (BE); Verstreken, Paul Clement, 3200 Aarschot (BE); Swennen, Jos, 3670 Meeuwen-Gruitrode (BE); Aegten, Jozef Theodoor, 3950 Bocholt (BE)
(74) Vertreter: Heraeus IP

(56) Entgegenhaltungen:
- EP-A2- 0 238 054
- EP-A2- 0 642 019
- DE-A1- 4 135 510
- DE-A1-102005 011 181
- US-A- 2 861 450
- US-A- 3 529 459
- US-A- 3 886 444
- US-A- 3 950 992
- US-A- 5 850 034
- US-B1- 6 216 526

## Beschreibung

Die Erfindung betrifft ein Verfahren zum Messen eines Gasgehaltes in einer Metallschmelze, wobei Gas in die Metallschmelze eingeleitet wird, dort in einen Gasaustausch mit in der Metallschmelze enthaltenem Gas tritt und anschließend aufgenommen und einer Messeinrichtung zur Auswertung zugeführt wird, wobei mindestens zwei unterschiedliche Gase in die Metallschmelze eingeleitet und ausgewertet werden, wobei beide Gase jeweils ein Trägergas aufweisen und eine Beimischung aus einem Gas, dessen Anteil in der Metallschmelze bestimmt werden soll.

Derartiges ist sind beispielsweise aus DE 10 2005 011 181 A1 oder aus EP 307 430 B1 bekannt. In den hier beschriebenen Vorrichtungen werden Gase aus einer Metallschmelze aufgesammelt und einer Messeinrichtung zugeführt, so dass der in der Metallschmelze enthaltene Gehalt an bestimmten Gasen gemessen werden kann. Dazu wird eine Gaszuleitung für die Zuleitung von Referenzgas bzw. Trägergas in die Metallschmelze durch den Gassammelkörper hindurch und an seiner Stirnseite aus ihm herausgeführt. Mit Hilfe der Gaszuleitung wird Referenzgas in die Metallschmelze eingeblasen. Das Referenzgas reichert sich mit in der Metallschmelze vorhandenen Gasen an oder, nach einer anderen Verfahrensweise, weist das Referenzgas eine höhere Konzentration des zu messenden Gases auf als die Metallschmelze, so dass das entstehende Gasgemisch eine geringere Konzentration des zu messenden Gasbestandteils aufweist als das Referenzgas. Das entstehende Gasgemisch wird von dem Gassammelkörper aufgenommen, durch die Gasableitung der Messeinrichtung zugeführt und ausgewertet. Im Detail ist das Messverfahren beispielsweise in EP 307 430 B1 beschrieben. Auch in EP 563 447 A1 sind derartige Messverfahren beschrieben.

Ähnliche Vorrichtungen und Verfahren sind aus US 6,216,526 B1 und aus EP 295 798 A1 bekannt.

Aufgabe der vorliegenden Erfindung ist es, die Effizienz des Messverfahrens zu erhöhen.

Die Aufgabe wird durch die Merkmale des unabhängigen Anspruchs gelöst. Vorzugsweise Ausgestaltungen ergeben sich aus den Unteransprüchen.

Das erfindungsgemäße Messverfahren zum Messen eines Gasgehaltes in einer Metallschmelze, wobei Gas in die Metallschmelze eingeleitet wird, dort in einen Gasaustausch mit in der Metallschmelze enthaltenem Gas tritt und anschließend aufgenommen und einer Messeinrichtung zur Auswertung zugeführt wird, wobei mindestens zwei unterschiedliche Gase in die Metallschmelze eingeleitet und ausgewertet werden, wobei beide Gase jeweils ein Trägergas aufweisen und eine Beimischung aus einem Gas, dessen Anteil in der Metallschmelze bestimmt werden soll erfolgt dadurch, dass die Konzentration des beigemischten Gases in jedem Fall der Gaseinleitung oberhalb der Konzentration des zu messenden Gases in der Metallschmelze liegt, und das die Konzentrationen des beigemischten Gases in jedem Fall der Gaseinleitung verschieden voneinander sind. Dabei wird von der vermutlichen Gaskonzentration in der Metallschmelze ausgegangen und für das einzuleitende Gas eine Konzentration deutlich über der erwarteten Konzentration in der Metallschmelze gewählt.

Es erfolgt dann in der Metallschmelze bei beiden Gasen entweder eine Absorption oder eine Desorption des zu messenden Gases. Es wird also mit zwei (oder mehr) Gasen gemessen, die unabhängig voneinander sind.

Dabei können die gleichen oder unterschiedliche Trägergase verwendet werden. Die in die Schmelze eingeleiteten Gase nehmen Gas aus der Schmelze auf, wenn die Konzentration des zu bestimmenden Gases in der Metallschmelze höher ist als die Konzentration dieses Gases in dem eingeleiteten Gas, so dass als eingeleitetes Gas auch reines Trägergas verwendet werden und die Konzentration des zu messenden Gases in dem eingeleiteten Gas Null sein kann. Im umgekehrten, erfindungsgemäßen Fall nimmt die Metallschmelze Gas aus dem eingeleiteten Gas auf, da in jedem Fall naturgemäß ein Gleichgewicht angestrebt wird. Zur Messung kann der Umstand genutzt werden, dass die Absorptions- und die Desorptionscharakteristiken von unterschiedlichen Gasen in Metallschmelzen unterschiedlich sein können.

Als Trägergas können Inertgase verwendet werden, vorzugsweise Argon und/oder Stickstoff. Als beigemischtes Gas kann Kohlenmonoxid verwendet werden, so dass der KohlenmonoxidGehalt in der Metallschmelze gemessen werden kann.

Eine zur Ausübung der Erfindung geeignete Vorrichtung zum Sammeln von Gasen in Metallschmelzen mit einem einen Sammelkörper aufweisenden Eintauchende, einer an dem Eintauchende mündenden Gaszuleitung und einer Gasableitung für die den Sammelkörper durchdringenden Gase, wobei der Gassammelkörper eine am Eintauchende angeordnete Stirnseite und Seitenwände aufweist, wobei zumindest ein Teil des Gassammelkörpers eine gasundurchlässige Schicht aufweist. Dadurch ist es möglich, einen größeren Teil der Gase durch den Gassammelkörper aufzunehmen und der Gasableitung und damit der Messeinrichtung zuzuführen, da die in den Gassammelkörper eindringenden Gase den Gassammelkörper zumindest im Wesentlichen nicht mehr außerhalb der Gasableitung verlassen können, so dass ein deutlich größerer Anteil der in dem Gassammelkörper aufgenommenen Gase der Messeinrichtung zugeführt werden kann. Dadurch wird die Messung einfacher, schneller und letztendlich auch genauer. Zweckmäßig ist es, dass mindestens ein Teil der äußeren Seitenwände eine gasundurchlässige Schicht aufweist. Der Gassammelkörper selbst kann an seiner Stirnseite einen bereits aus dem Stand der Technik (s. oben) bekannten Hohlraum aufweisen. In diesem Hohlraum sammeln sich zunächst die aus der Schmelze kommenden Gase. Sie dringen dann in den Gassammelkörper ein, da sie aus dem Hohlraum nicht anders entweichen können. Durch die seitliche Abschirmung durch die gasundurchlässige Schicht können die Gase nur in die Gasableitung entweichen. Dazu kann die gasundurchlässige Schicht an der Oberfläche die Seitenwände des Gassammelkörpers angeordnet sein. Vorteilhaft ist es, dass die Schicht aus mindesten zwei aufeinander angeordneten Teilschichten gebildet ist. Die untere, dem Inneren des Gassammelkörpers zugewandte Teilschicht kann aus Metall, insbesondere aus einem Metall mit höherem Schmelzpunkt als Eisen gebildet sein. Als Metalle kommen insbesondere in Frage Molybdän, Titan, Vanadium, Chrom, Niob oder eine Legierung mit mindestens einem dieser Metalle. Die untere, innere Teilschicht ist gasdicht. Auf ihr kann eine äußere, dem Inneren des Gassammelkörpers abgewandte Teilschicht aus Keramik aufgebracht sein. Diese kann als Schutzschicht für die zwischen ihr und dem Gassammelkörper angeordnete untere Teilschicht aus Metall wirken. Die äußere Teilschicht kann vorzugsweise aus Oxidkeramik oder einem Silikat, insbesondere aus Zirkoniumdioxid, Aluminiumoxid, Chromdioxid, Zirkoniumsilikat, Aluminiumsilikat oder Spinell gebildet sein. Der Gassammelkörper kann mit der Schicht nahezu vollständig umgeben sein, wobei lediglich der stirnseitige Gaseintritt in den Gassammelkörper und der Zugang zur Gasableitung aus dem Gassammelkörper unbeschichtet sind. Sinnvoll ist es, die gesamte Stirnseite des Gassammelkörpers beschichtungsfrei zu lassen, oder auch nur die Oberfläche des stirnseitigen Hohlraumes des Gassammelkörpers. Vorzugsweise ist mindestens eine der Teilschichten plasmagespritzt. Zweckmäßigerweise kann der Gassammelkörper eine zylindrische oder konische Seitenwand aufweisen. Die Gasableitung ist vorzugsweise an der der Stirnseite gegenüber liegenden Rückwand des Gassammelkörpers angeordnet. Die Gasableitung kann beispielsweise an einem Gasanschlussstutzen oder in einer Öffnung des Gassammelkörpers angeordnet sein.

Die Vorrichtung wird zum Messen des Gasgehaltes in einer Metallschmelze verwendet. Es sind Messungen zum Beispiel in den unterschiedlichsten Stahlschmelzen möglich. Der Gassammelkörper selbst ist undurchlässig für die Metallschmelze, aber sehr gut gasdurchlässig und aufnahmefähig für die zu messenden Gase.

Ein Ausführungsbeispiel der Erfindung wird nachfolgend an Hand einer Zeichnung näher erläutert.

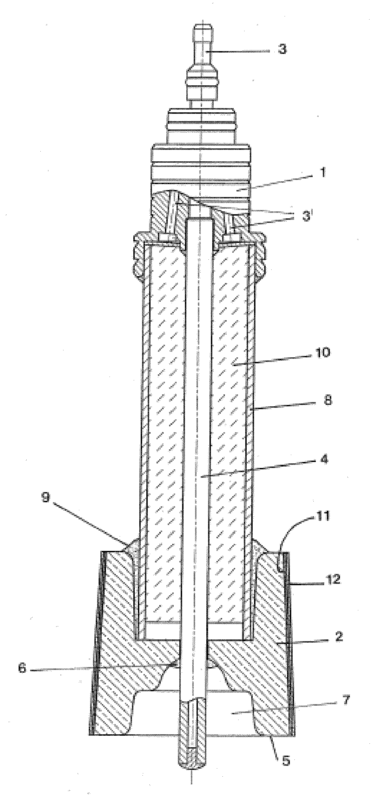

Die Zeichnung zeigt eine Vorrichtung, teilweise geschnitten.

Die in der Zeichnung dargestellte Vorrichtung wird an einem Befestigungsstutzen 1 an einem nicht dargestellten Trägerrohr befestigt und mit diesem in eine Stahlschmelze eingetaucht. In die Stahlschmelze wird der Gassammelkörper 2 getaucht, um dort den Gasaustausch vorzunehmen.

In dem Befestigungsstutzen 1 sind Gasanschlüsse 3; 3' angeordnet. Dabei mündet der zentrale Gasanschluss 3 in die zentrisch in der Vorrichtung angeordnete Gaszuleitung 4. Diese ist zentrisch durch den Gassammelkörper hindurch geführt und endet unterhalb der Stirnseite 5 des Gassammelkörpers. Durch die Gaszuleitung 4 wird Trägergas in die Metallschmelze eingeleitet. Die Gaszuleitung 4 besteht im Wesentlichen aus einem Quarzrohr, welches an seinem Eintauchende gebogen sein kann, so dass die Mündungsöffnung in Richtung des Gassammelkörpers 2 gerichtet ist. Die Gaszuleitung 4 ist in dem Gassammelkörper 2 mittels Zement 6 fixiert. Das durch die Gaszuleitung 4 in die Metallschmelze einströmende Trägergas nimmt aus der Metallschmelze Gase auf, steigt in den Hohlraum 7 des Gassammelkörpers 2 und dringt von dort und von der Stirnseite 5 her in den Gassammelkörper 2 ein. Dieser ist aus einem porösen Material gebildet, beispielsweise aus Zement. Auch ein Keramikkörper, beispielsweise Aluminiumoxid, ist möglich. Durch die Poren des Gassammelkörpers dringt das Gas nach oben in die Gasableitung. Diese ist im Wesentlichen gebildet aus einem Quarzglasrohr 8, welches mittels Zement 9 in dem Gassammelkörper 2 fixiert ist. In dem Quarzglasrohr ist eine poröse Füllung 10 aus Aluminiumoxid, zum Beispiel in Kugelform, angeordnet. Durch die Füllung 10 erfolgt die Ableitung des mit Gas aus der Metallschmelze gemischten Trägergases durch die Gasanschlüsse 3' zu einer Messeinrichtung. Dort wird das entnommene Gas mit dem in die Metallschmelze eingeleiteten Gas verglichen und so das aus der Schmelze aufgenommene (oder abgegebene) Gas ausgewertet und dadurch der Gasgehalt in der Metallschmelze bestimmt. Dieser Vorgang ist an sich hinreichend bekannt und beispielsweise in EP 307 430 B1 (oder ähnlich in EP 563 447 A1) beschrieben. Als Trägergas des eingeleiteten Gases wird Argon verwendet. Dem Trägergas wird zur Messung des Kohlenmonoxidgehaltes in der Stahlschmelze jeweils Kohlenmonoxid mit einem Anteil von jeweils mehr als 2,5 % (beispielsweise von 5 % und 10 %) beigemischt, da der erwartete Gasgehalt bei 2,5 % liegt.

Der Gassammelkörper 2 weist an seiner konischen Außenfläche eine aus einer unteren Teilschicht 11 und einer äußeren Teilschicht 12 bestehende gasundurchlässige Schicht auf. Die untere Teilschicht 11 ist aus Molybdän gebildet, die äußere Teilschicht 12 dient als Schutzschicht und ist aus Spinell gebildet.

Prinzipiell kann die gasundurchlässige Schicht auch an dem Eintauchende angewandten Ende des Gassammelkörpers 2 angeordnet sein. Dies ist jedoch im Regelfall nicht nötig, da die dort vorhandenen Oberflächen derart klein sind, dass ein Gasaustritt nur in nicht nennenswertem Umfang erfolgt. Dadurch wird praktisch das gesamte von der Vorrichtung aufgenommene Gas in die von dem Quarzglasrohr 8 begrenzte Gasableitung geleitet.

Mit der Vorrichtung kann auch der Gehalt an Wasserstoff oder Stickstoff in Stahlschmelzen bestimmt werden.

## Patentansprüche

1. Verfahren zum Messen eines Gasgehaltes in einer Metallschmelze, wobei Gas in die Metallschmelze eingeleitet wird, dort in einen Gasaustausch mit in der Metallschmelze enthaltenem Gas tritt und anschließend aufgenommen und einer Messeinrichtung zur Auswertung zugeführt wird, wobei mindestens zwei unterschiedliche Gase in die Metallschmelze eingeleitet und ausgewertet werden, wobei beide Gase jeweils ein Trägergas aufweisen und eine Beimischung aus einem Gas, dessen Anteil in der Metallschmelze bestimmt werden soll, **dadurch gekennzeichnet, dass** die Konzentration des beigemischten Gases in jedem Fall der Gaseinleitung oberhalb der Konzentration des zu messenden Gases in der Metallschmelze liegt, und dass die Konzentrationen des beigemischten Gases in jedem Fall der Gaseinleitung verschieden voneinander sind.

2. Verfahren nach Anspruch 1, **dadurch gekennzeichnet, dass** die eingeleiteten Gase unterschiedliche Trägergase aufweisen.

3. Verfahren nach Anspruch 1 oder 2, **dadurch gekennzeichnet, dass** als Trägergas ein Inertgas, insbesondere Argon und/oder Stickstoff, verwendet wird.

4. Verfahren nach einem der Ansprüche 1 bis 3, **dadurch gekennzeichnet, dass** das beigemischte Gas Kohlenmonoxid ist.

## Claims

1. Process for measuring a gas content in a molten metal, whereby gas is introduced into the molten metal, where it engages in a gas exchange with gas contained in the molten metal and subsequently is taken up and supplied to a measuring facility for analysis, whereby at least two different gases are introduced into the molten metal and are analysed, whereby both gases comprise a carrier gas each and an admixture of a gas whose fraction in the molten metal is to be determined, **characterised in that** the concentration of the admixed gas, in any case of gas being introduced, is above the concentration of the gas in the molten metal to be measured, and **in that** the concentrations of the admixed gas, in any case of gas being introduced, differ from each other.

2. Process according to claim 1, **characterised in that** the introduced gases comprise different carrier gases.

3. Process according to claim 1 or 2, **characterised in that** an inert gas, in particular argon and/or nitrogen, is used as carrier gas.

4. Process according to any one of the claims 1 to 3, **characterised in that** the admixed gas is carbon monoxide.

## Revendications

1. Procédé de mesure d'une teneur en gaz dans une masse fondue métallique, dans lequel du gaz est introduit dans la masse fondue métallique, y entre dans un échange gazeux avec du gaz contenu dans la masse fondue métallique et est ensuite absorbé et amené à un dispositif de mesure pour analyse, dans lequel au moins deux gaz différents sont introduits dans la masse fondue métallique et analysés, dans lequel les deux gaz présentent chacun un gaz porteur et un additif constitué d'un gaz dont la proportion dans la masse fondue métallique doit être déterminée, **caractérisé en ce que** la concentration du gaz ajouté dans chaque cas de l'introduction de gaz se situe au-dessus de la concentration du gaz à mesurer dans la masse fondue métallique, et que les concentrations du gaz ajouté dans chaque cas de l'introduction de gaz sont différentes les unes des autres.

2. Procédé selon la revendication 1, **caractérisé en ce que** les gaz introduits présentent différents gaz porteurs.

3. Procédé selon la revendication 1 ou 2, **caractérisé en ce qu'**un gaz inerte, notamment de l'argon et/ou de l'azote, est utilisé comme gaz porteur.

4. Procédé selon une des revendications 1 à 3, **caractérisé en ce que** le gaz ajouté est du monoxyde de carbone.
